# EUROPÄISCHE PATENTSCHRIFT

(11) **EP 0 909 954 B1**
(45) Veröffentlichungstag und Bekanntmachung des Hinweises auf die Patenterteilung: **26.11.2003**
(21) Anmeldenummer: 98119255.2
(22) Anmeldetag: 13.10.1998
(51) Int. Cl.: G01N 33/68, G01N 33/577

(54) **Nachweis von Knorpelerkrankungen mit MIA**
Detection of cartilage diseases by MIA
Détection des maladies des cartilages par MIA

(30) Priorität: 16.10.1997 US 62234 P
(43) Veröffentlichungstag der Anmeldung: 21.04.1999
(73) Patentinhaber: Scil Technology Holding GmbH, 82152 Martinsried (DE)
(72) Erfinder: Bosserhoff, Anja-Katrin, Dr., 93059 Regensburg (DE); Büttner, Reinhard, Dr., 93090 Bach/Donau (DE); Kaufmann, Martin, 82362 Weilheim (DE)
(74) Vertreter: Böhm, Brigitte, Dipl.-Chem. Dr.

(56) Entgegenhaltungen:
- EP-A- 0 085 415
- WO-A1-89/07764
- WO-A1-91/19001
- DE-A1- 19 653 358

## Beschreibung

Gegenstand der vorliegenden Erfindung ist ein Verfahren zur Diagnose von Knorpelerkrankungen durch den Nachweis von MIA, ein dafür geeignetes Reagenz, sowie die Verwendung von Antikörpern gegen MIA zum Nachweis von Knorpelerkrankungen.

Knorpelerkrankungen und insbesondere degenerative Knorpelerkrankungen können auf verschiedene Ursachen zurückzuführen sein. Diese Erkrankungen von Knorpel und Gelenken werden oftmals als Arthritis bezeichnet. Die Einteilung der Arthritis-Formen kann nach Anzahl der betroffenen Gelenke (Mono-, Oligo-, Polyarthritis), nach deren Verlauf (akut, subakut, chronisch) oder deren Ursache (autoimmun, entzündlich, etc) erfolgen. Arthritis kann beispielsweise durch Infektion mit Bakterien oder Viren, aufgrund von Entzündungsvorgängen, bei Erkrankungen des Gelenkknorpels (Arthrosen), aufgrund von Stoffwechselerkrankungen wie Gicht, oder aufgrund von Autoimmunerkrankungen wie Rheuma (rheumathoide Arthritis) oder systemischem Lupus erythematodes entstehen. Neben der rheumatoiden Arthritis, die häufig mit erhöhten Entzündungsparametern einhergeht, ist vor allem die Osteoarthritis zu beachten, die meist ohne erkennbares Entzündungsgeschehen abläuft.

Im Stand der Technik existieren bisher nur einige unspezifische oder kostenintensive Diagnose-Verfahren zum Nachweis von Knorpelerkrankungen wie beispielsweise Arthritis. Rheumatoide Arthritis wird beispielsweise derzeit zum einen aufgrund der Symptomatik (geschwollene Gelenke, Schmerzen an den Gelenken, Bewegungsunfähigkeit) diagnostiziert, zum anderen durch den Nachweis von Rheumafaktoren, den Nachweis des klassischen Akute-Phase-Proteins CRP (C-reaktives Protein), den Nachweis von antinukleären Antikörpern (ANA) oder Bestimmung der Blutsenkungsgeschwindigkeit (BSG). Als teure physikalische Methoden kommen Röntgenuntersuchungen oder Kernspintomographie zum Einsatz.

Allen zuvor genannten biochemischen Nachweisverfahren bzw. den nachzuweisenden Parametern ist gemeinsam, daß sie generell bei Entzündungen erhöht und somit unspezifisch sind. Wegen der Unspezifität dieser Tests ist eine Differenzierung von Knorpelerkrankungen und insbesondere degenerativen Knorpelerkrankungen gegenüber anderen Erkrankungen entzündlicher und nicht-entzündlicher Natur nicht möglich. Die Rheumafaktorkonzentration ist beispielweise auch erhöht bei einem Teil der Patienten mit Hepatitis, Sarkoidose und verschiedenen Infektionskrankheiten, sowie bei anderen Gelenkerkrankungen wie dem Reiter-Syndrom. Ein Konzentrationsanstieg von CRP im Plasma oder eine erhöhte Blutsenkungsgeschwindigkeit weisen lediglich generell auf ein entzündliches Geschehen hin. Eine erhöhte Konzentration von antinukleären Antikörpern zeigt nur an, daß eine Autoimmunkrankheit vorliegt.

Für den Nachweis von Osteoarthritis werden derzeit vorwiegend bildgebende Verfahren eingesetzt. Ein immundiagnostischer spezifischer Nachweis für Osteoarthritis existiert bisher nicht.

In der WO 91/19001 ist ein immundiagnostisches Verfahren zum Nachweis von rheumatoider Arthritis durch den Einsatz von Antikörpern gegen einen Komplex aus IgA und α1-Antitrypsin beschrieben. Das α1-Antitrypsin korreliert jedoch oft mit generellen Entzündungsparametem. Der Komplex mit IgA ist ist ebenfalls vor allem bei entzündlichen Prozessen erhöht. Eine spezifische Diagnose der rheumatoiden Arthritis ist somit nicht möglich.

In der EP-B 0 363 449 wird ein spezifisches ANA-Antigen von etwa 33 kDa beschrieben, das mit Antikörpern in Proben von Patienten mit chronischer Polyarthritis reagiert. Die dort beschriebenen Autoantikörper werden nur in einem Teil der Patienten mit chronischer Polyarthritis gefunden und treten auch bei einigen anderen Autoimmunerkrankungen auf. Zudem ist der Titer dieser Autoantikörper meist nicht mit der Schwere der Erkrankung korreliert.

Die beiden zitierten Patentanmeldungen beschreiben exemplarisch die diagnostischen Probleme: Bei den serologischen Tests (Autoantikörperbestimmungen) mangelt es sowohl an der Sensitivität als auch an der Spezifität, sowie einer Aussage zur Schwere der Erkrankung. Bei den Parametern/Verfahren zur Diagnose einer rheumatoiden Arthritis handelt es sich meist um Ansätze, die mit den unspezifischen Entzündungsmarkern korrelieren und somit nicht die diagnostisch erforderliche Spezifität aufweisen. Die Knorpeldestruktion ist mit den im Stand der Technik beschriebenen Verfahren nicht direkt nachzuweisen.

Im Stand der Technik ist bisher kein diagnostisches Verfahren beschrieben, mit dem sensitiv, frühzeitig, zuverlässig und spezifisch Knorpelerkrankungen und insbesondere degenerative Knorpelerkrankungen wie Arthritis, insbesondere rheumatoide Arthritis und Osteoarthritis nachgewiesen werden können.

Aufgabe war es daher, ein verbessertes diagnostisches Verfahren zum Nachweis von Knorpelerkrankungen und insbesondere degenerativen Knorpelerkrankungen zu entwickeln, mit welchem die Nachteile des Standes der Technik zumindest teilweise überwunden werden. Mit Hilfe des Verfahrens sollte eine Differenzierung von Knorpelerkrankungen und insbesondere degenerativen Knorpelerkrankungen gegenüber anderen Erkrankungen ermöglicht werden.

Die Aufgabe wird gelöst durch die Bereitstellung eines diagnostischen Verfahrens zum Nachweis eines Proteins mit der Bezeichnung MIA. Das Verfahren ist dadurch gekennzeichnet, daß MIA (melanoma inhibiting activity, Melanom inhibierende Aktivität) nachgewiesen wird.

MIA ist ein ca. 11 kDa großes, lösliches Protein, das von malignen Melanomzellen sekretiert wird. Das maligne Melanom ist ein von den pigmentbildenden Zellen der Haut, den Melanocyten, ausgehender bösartiger Tumor, der stark zur Metastasenbildung neigt. MIA ist seit kurzem als Serummarker für das maligne Melanom bekannt (Bosserhoff et al, Cancer Research 57, 1997, S. 3149-3153).

Überraschenderweise hat sich herausgestellt, daß MIA, obwohl ursprünglich als Melanomassoziertes Protein beschrieben, sich ausgezeichnet als spezifischer Marker für Knorpelerkrankungen, vor allem degenerative Knorpelerkrankungen und dabei insbesondere Arthritis eignet. Das erfindungsgemäße Verfahren ist dadurch gekennzeichnet, daß MIA nachgewiesen wird. Bevorzugt wird MIA immunologisch nachgewiesen.

Es hat sich gezeigt, daß MIA im Vergleich zu den Parametern des Standes der Technik ein deutlich spezifischerer Parameter für Knorpelerkrankungen und insbesondere degenerative Knorpelerkrankungen wie zum Beispiel Arthritis ist. Eine gegenüber einer gesunden Patientenprobe erhöhte Konzentration von MIA ist ein starker Hinweis auf das Vorliegen einer Arthritis.

Da MIA ein Produkt der Chondrozyten, das sind in die Knorpelmatrix eingelagerte Knorpelzellen, darstellt, ist die Konzentration an MIA bei Arthritis-negativen Proben (z.B. bei Entzündungsprozessen, die nicht am Knorpel ablaufen, nicht oder kaum erhöht. Dies bedeutet einen erheblichen Fortschritt gegenüber dem Stand der Technik. Die bisher bekannten Parameter sind für degenerative Knorpelerkrankungen nicht spezifisch, sondern können auch bei Entzündungen anderer Genese oder bei Infektionen erhöht sein.

Durch den Nachweis von MIA ist es möglich, die Zerstörung des Knorpels bzw. des Gelenks direkt zu beobachten.

Durch den Nachweis von MIA werden falsch positive Nachweisreaktionen weitestgehend vermieden.

Zu den bevorzugt nachgewiesenen degenerativen Knorpelerkrankungen gehören rheumatoide Arthritis, HLA B27 (assoziierte Arthritis) und Gicht. Als besonders geeignet hat sich das erfindungsgemäße Verfahren zum Nachweis von rheumatoider Arthritis erwiesen. Denkbar für den Nachweis durch das erfindungsgemäße Verfahren ist allerdings auch der Nachweis von anderen Knorpel- und Gelenkerkrankungen wie beispielsweise Osteoarthritis, Psoriasis Arthritis, systemischem Lupus erythematodes, systemischer Sklerose oder Relapsing Polychondritis.

Das erfindungsgemäße Nachweisverfahren kann in allen dem Fachmann geläufigen Testformaten zum Nachweis eines Proteins verwendet werden. Neben den sogenannten Naßtesten, bei denen die Testreagenzien in flüssiger Phase vorliegen, können auch alle gängigen Trockentestformate, die zum Nachweis von Proteinen bzw. Antikörpern geeignet sind, verwendet werden. Bei diesen Trockentests oder Teststreifen, wie sie beispielsweise in der BP-A-0 186 799 beschrieben sind, sind die Testkomponenten auf einem Träger aufgebracht. Wird das erfindungsgemäße Verfahren im Teststreifenformat durchgeführt, ist daher kein Wasch-Schritt notwendig. Bevorzugt wird das erfindungsgemäße Verfahren jedoch als Naßtest durchgeführt.

Zu den in Frage kommenden Testführungen gehören heterogene Formate, bei denen eine Festphase beteiligt ist, sowie homogene Formate.

Bevorzugt werden jedoch heterogene Testformate verwendet. Dabei wird mindestens ein Rezeptor R1 eingesetzt, der mit dem Analyten MIA spezifisch bindefähig und an eine Festphase gebunden oder mit einer Festphase bindefähig ist. Die Probe wird mit R1 zusammen inkubiert. Dabei kann die Festphase bereits vorhanden sein oder später zugegeben werden. Zum Nachweis des gebildeten Komplexes aus R1 und Analyt kann ein weiterer Rezeptor R2 dazugegeben werden, der eine Markierung trägt. R2 ist entweder mit dem Analyten oder mit dem Rezeptor R1 spezifisch bindefähig.

Wenn R2 mit dem Analyten spezifisch bindefähig ist, so entsteht ein Sandwich-Komplex aus R1-Analyt-R2, der an der Festphase gebunden ist. Nach der Trennung der festen von der flüssigen Phase wird die Markierung in einer der beiden Phasen bestimmt. Bei der Durchführung des Sandwich-Verfahrens ist R2 ein Antikörper. Dieses Sandwich-Verfahren wird bevorzugt für den diagnostischen MIA-Nachweis für Arthritis eingesetzt.

Beim Sandwich-Test kann der Test einstufig, d. h. alle Komponenten sind gleichzeitig anwesend, durchgeführt werden. Diese vereinfachte Testführung ist insbesondere bei Screening-Tests, bei denen eine Vielzahl von Proben in möglichst rascher Folge getestet werden sollen, ein großer Vorteil. Ein solcher Screening-Test ist ebenfalls Gegenstand der Erfindung.
Wenn es sich bei R2 um einen Rezeptor handelt, der mit R1 spezifisch bindefähig ist, so verdrängt R2 den Analyten. Es handelt sich hierbei also um einen kompetitiven Test. Der Nachweis der Markierung erfolgt nach Trennung der festen von der flüssigen Phase in einer der beiden oder in beiden Phasen.

R 1 ist ein Rezeptor, der mit dem Analyten MIA spezifisch bindefähig und außerdem an eine Festphase gebunden werden kann. Es handelt sich hierbei bevorzugt um einen mit einer Festphase bindefähigen Antikörper.

Erfindungsgemäß werden unter der Bezeichnung "Antikörper" sowohl monoklonale als auch polyklonale Antikörper verstanden. Unter den Begriff "Antikörper" fällt sowohl der vollständige Antikörper als auch alle bei Immuntests und sonstigen Verwendungen gängigen Fragmente davon, wie F(ab')2-, Fab'- oder Fab-Fragmente. Umfaßt sind auch solche Antikörper, die durch Veränderung der Antikörper hergestellt wurden, solange die Antigenbindeeigenschaft nicht entscheidend beeinflußt wurde. Beispielsweise können durch gentechnologische Maßnahmen Teile der normalerweise in Mäusen hergestellten monoklonalen Antikörper durch entsprechende humane Antikörpersequenzen ersetzt werden, um unspezifische Bindungen im Immunoassay zu minimieren. Verfahren zur Herstellung solcher chimärer monoklonaler Antikörper sind dem Fachmann beispielsweise aus Antibody Engineering, J. Mc Cafferty, H.R. Hoogenboom und D.J. Chiswell, The Practical Approach Series, Series Editor: B.D. Hames, Oxford University Press, 1996, bekannt.

Im erfindungsgemäßen Verfahren werden als Bestandteil von R1 besonders bevorzugt monoklonale Antikörper gegen MIA eingesetzt.

R1 kann entweder direkt an die Festphase gebunden sein. Die direkte Bindung von R1 an die Festphase erfolgt nach dem Fachmann bekannten Methoden. Die Bindung von R1 an die Festphase kann auch indirekt über ein spezifisches Bindungssystem erfolgen. In diesem Fall ist R1 ein Konjugat, das aus einem Rezeptor, bevorzugt einem Antikörper, und einem Reaktionspartner eines spezifischen Bindungssystems besteht. Unter einem spezifischen Bindungssystem werden hier zwei Partner verstanden, die spezifisch miteinander reagieren können. Das Bindungsvermögen kann dabei auf einer immunologischen Reaktion oder auf einer anderen spezifischen Reaktion beruhen. Bevorzugt wird als spezifisches Bindungssystem eine Kombination von Biotin und Avidin oder Biotin und Streptavidin verwendet. Weitere bevorzugte Kombinationen sind Biotin und Antibiotin, Hapten und Anti-Hapten, Fc-Fragment eines Antikörpers und Antikörper gegen dieses Fc-Fragment oder Kohlenhydrat und Lectin. Einer der Reaktionspartner dieses spezifisch bindefähigen Paares ist dann Teil des Konjugates, das den Rezeptor R1 bildet.

Der andere Reaktionspartner des spezifischen Bindungssystems liegt in fester Phase vor. Die Bindung des anderen Reaktionspartners des spezifischen Bindungssystems, bevorzugt Streptavidin, an ein unlösliches Trägermaterial kann nach den üblichen, dem Fachmann bekannten Methoden vorgenommen werden. Hierbei ist sowohl eine kovalente als auch eine adsorptive Bindung geeignet. Als feste Phase besonders geeignet sind Reagenzgläschen oder Mikrotiterplatten aus Polystyrol oder ähnlichen Kunststoffen, die an der Innenoberfläche mit Reaktionspartner des spezifischen Bindungssystems beschichtet sind Weiterhin geeignet und besonders bevorzugt sind teilchenförmige Substanzen, wie beispielsweise Latexpartikel, Molekularsiebmaterialien, Glaskörperchen, Kunststoffschläuche und dergleichen. Auch poröse, schichtförmige Träger wie Papier können als Träger verwendet werden.

R2 besteht aus einem Rezeptor, der spezifisch für den Analyten MIA oder für R1 ist, und einer Markierung. Ist R2 spezifisch für den Analyten, so besteht R2 bevorzugt aus einem Antikörper, der für MIA spezifisch ist und einer Markierung. Ist R2 spezifisch für R1, so besteht R2 bevorzugt aus dem Analyten MIA oder einem Analogon und einer Markierung.

Im erfindungsgemäßen Verfahren werden als Bestandteil von R2 besonders bevorzugt monoklonale Antikörper gegen MIA eingesetzt.

Bevorzugt wird als Markierung von R2 eine direkt nachweisbare Substanz, beispielsweise eine chemilumineszierende, fluoreszierende oder radioaktive Substanz oder ein Metallsol-, Latex- oder Goldpartikel eingesetzt. Weiterhin bevorzugt als Markierung sind Enzyme oder andere biologische Moleküle wie beispielsweise Haptene. Unter den Haptenen ist Digoxigenin eine besonders bevorzugte Markierung. Unter den Enzymen gehört Peroxidase zu den bevorzugten Markierungen. Die Verfahren zur Markierung sind dem Fachmann geläufig und bedürfen hier keiner weiteren Erklärung. Der Nachweis der Markierung erfolgt in an sich bekannter Weise direkt durch Messung der chemilumineszierenden, fluoreszierenden oder radioaktiven Substanz oder des Metallsol-, Latex- oder Goldpartikels oder durch Messung des durch das Enzym umgesetzten Substrats.

Der Nachweis der Markierung kann auch auf indirekte Weise erfolgen. Dabei bindet ein weiterer Rezeptor, der selbst wiederum mit einer signalerzeugenden Gruppe gekoppelt ist, spezifisch an die Markierung von R2, bespielsweise ein Hapten wie Digoxigenin. Der Nachweis der signalerzeugenden Gruppe, beispielsweise eine chemilumineszierende, fluoreszierende oder radioaktive Substanz oder ein Enzym oder Goldpartikel, erfolgt nach dem Fachmann geläufigen Methoden. Als weiterer Rezeptor kann beispielsweise ein Antikörper oder ein Antikörperfragment eingesetzt werden, der spezifisch an die Markierung von R2 bindet. Wird dieser indirekte Nachweis der Markierung verwendet, so ist die Markierung von R2 bevorzugt Digoxigenin oder ein anderes Hapten, und der Nachweis erfolgt über einen mit Peroxidase gekoppelten Antikörper, der gegen Digoxigenin oder gegen das Hapten gerichtet ist.

Als Proben können alle dem Fachmann bekannten biologischen Flüssigkeiten verwendet werden. Bevorzugt werden als Probe Körperflüssigkeiten wie Vollblut, Blutserum, Blutplasma, Synovialflüssigkeit (Gelenkflüssigkeit), Lymphflüssigkeit, Urin, Speichel etc. eingesetzt.

In den Testansätzen können neben der Probe, der Festphase und den aufgeführten Rezeptoren weitere, je nach Anwendung benötigte Zusätze wie Puffer, Salze, Detergenzien, Proteinzusätze wie beispielsweise RSA vorhanden sein. Die notwendigen Zusätze sind dem Fachmann bekannt oder können von ihm auf einfache Weise herausgefunden werden.

Ein weiterer Gegenstand der Erfindung ist ein Reagenz zum Nachweis von Knorpelerkrankungen und insbesondere degenerativen Knorpelerkrankungen durch den immunologischen Nachweis von MIA, das einen für MIA spezifischen, mit einer Festphase bindefähigen Rezeptor R1 und die üblichen Testzusätzen für Immunoassays wie Puffer, Salze, Detergenzien, Reduktionsmittel wie DTT etc. enthält.

Ebenfalls ein Gegenstand der Erfindung ist ein Reagenz zum Nachweis von Knorpelerkrankungen und insbesondere degenerative Knorpelerkrankungen durch den immunologischen Nachweis von MIA, das zusätzlich zu R1 einen Rezeptor R2 enthält, der für R1 oder den Analyten MIA spezifisch ist und eine Markierung trägt.

Ebenfalls Gegenstand dieser Erfindung ist die Verwendung von Antikörpern gegen MIA zum Nachweis von Knorpelerkrankungen und insbesondere degenerativen Knorpelerkrankungen.

Die erfindungsgemäßen monoklonalen Antikörper können in an sich bekannter Weise durch Immunisierung mit isoliertem MIA (aus humanem Gewebe isoliert oder rekombinant) in geeigneten Versuchstieren wie beispielsweise Mäuse, Ratten, Kaninchen und anschließender Fusion der Milzzellen der immunisierten Tiere mit Myelomzellen hergestellt werden. Neben Milzzellen als Lymphocyten-Quelle können auch periphere Blut-Lymphocyten (PBL) oder Lymphknoten-Zellen von immunisierten Tieren (bevorzugt: Maus oder Ratte) verwendet werden.

Alternativ können auch Lymphocyten von humanen Spendern (Gewebeproben von Melanom-Patienten), die Antikörper gegen MIA entwickelt haben, immortalisiert werden. Solche Anti-MIA-Antikörper produzierenden Lymphocyten können entweder durch Fusion mit einer humanen Myelom-Zellinie oder durch Epstein-Barr-Virus (EBV)-Transformation zu antikörper-produzierenden Hybridom-Zellen immortalisiert werden (Monoclonal Antibody and Immunosensor Technology, A.M. Campbell, Elsevier Verlag 1991; Monoklonale Antikörper, J.H. Peters, H. Baumgarten, Springer Verlag 1990; Monoclonal Antibody Production Techniques and Applications, ed. Lawrence B. Schook, Marcel Dekker Verlag 1987).

Das folgende Beispiel erläutert die Erfindung weiter.

### Beispiel 1

### ELISA-Test zum Nachweis von MIA zur Diagnose von Knorpelerkrankungen

### Inkubationspuffer des MIA-ELISAs (Gewichtsangaben / Liter):

| | |
|---|---|
| NaH₂PO₄ | 0.97 g |
| Na₂HPO₄ | 5.88 g |
| Di-Na-Tartrat | 46 g |
| Synperonic F 68 | 5 g |
| Rinder-IgG | 1 g |
| Rinder-Albumin | 2 g; der pH-Wert wird mit NaOH ad 7.4 eingestellt. |

### Durchführung:

In die Vertiefungen einer mit Streptavidin beschichteten Mikrotiterplatte (Boehringer Mannheim GmbH, Id.Nr. 1 664 778) werden je 10 µl Standardlösung (rec.h-MIA in 50 mmol/l Hepes, 150 mmol/l NaCl, pH 7.0, 2 % w/v BSA; Verdünnungsreihe mit 0, 3.13, 6.25, 12.5, 25, 50 ng/ml) oder 10 µl der zu bestimmenden Probe ( Serum, Plasma etc.) vorgelegt. In jede mit Standard oder Probe beschickte Vertiefung werden anschließend 200 µl einer Lösung von 1 µg/ml MAK<MIA>M-2F7-Bi und 25 mU/ml MAK<MIA>M-1A10-POD in Inkubationspuffer pipettiert. Die Mikrotiterplatte wird dann 45 Minuten bei Raumtemperatur (20 - 25°C) auf einem handelsüblichen Schüttelgerät für Mikrotiterplatten (z. B. IKA MTS 4) bei 500 rpm geschüttelt.

Die Vertiefungen der Mikrotiterplatte werden sorgfältig entleert und dreimal mit je 300 µl Waschlösung (250 mg NaCl/l, 1 mg CuSO₄/l, Boehringer Mannheim GmbH, Id.Nr. 1 059 475) gewaschen.

In jede der beschichteten Vertiefungen der Mikrotiterplatte werden 200 µl ABTS®-Substratlösung ( 2,2'- Azino-di[3-ethylbenzthiazolin-sulfonat] 1.9 mmol/l ABTS®, 100 mmol/l Phosphat-Citrat-Puffer, pH 4.4, Natriumperborat 3.2 mmol/l) pipettiert und 30 Minuten bei Raumtemperatur (20 - 25°C) auf dem Plattenschüttler bei 500 rpm inkubiert.

Die Extinktionswerte der sich ergebenden Farblösungen in den Vertiefungen werden mit einem für Mikrotiterplatten geeigneten Photometer bei 405 nm gemessen. Aus den Signalen und den Konzentrationswerten der Standardreihe wird eine Standardkurve erstellt, an der die Probenkonzentrationen abgelesen werden.

Mit dem hier beschriebenen ELISA-Testverfahren wurden die MIA-Konzentrationen im Serum von Patienten mit rheumatoider Arthritis (RA), HLA B27-assoziierter Oligoarthritis (HLA B27), Gicht und von gesunden Normalpatienten (NP) bestimmt.

In Tabelle 1 sind die Ergebnisse dargestellt.

**Tabelle 1**

| **Art der Proben** | **Anzahl der getesteten Proben** | **Anteil d. Proben mit erhöhter MIA-Konz.** | **durchschnittliche MIA-Konzentration in ng/ml** |
|---|---|---|---|
| Normal-Patienten | 100 | 0 | 0 - 4; cutoff: 6,5 |
| RA | 46 | 24/46 = 52 % | 12,3 |
| HLA B27 | 12 | 6/12 = 50 % | 6,6 |
| Gicht | 10 | 4/10 = 40 % | 6,6 |

Es zeigt sich, daß erhöhte MIA-Serumkonzentrationen in Patienten mit degenerativen Knorpelerkrankungen, die mit Gelenkszerstörung assoziiert sind, gefunden werden konnten. Insbesondere beim Vorliegen rheumatischer Arthritis (RA) ist die MIA-Konzentration deutlich erhöht. Als Cutoff-Wert wurde eine MIA-Konzentration von 6,5 ng/ml bestimmt.

Die höchsten MIA-Konzentrationen waren mit positiven Ergebnissen für Rheumafaktoren assoziiert. Die höchsten MIA-Konzentrationen waren jedoch nicht mit einer erhöhten Blutsenkungsgeschwindigkeit oder erhöhter CRP-Konzentration korreliert.

Dies zeigt, daß MIA als diagnostischer Marker für Knorpelerkrankungen und dabei vor allem für degenerative Knorpelerkrankungen, insbesondere rheumatische Arthritis geeignet ist.

## Patentansprüche

1. Verfahren zur Diagnose von Knorpelerkrankungen, **dadurch gekennzeichnet, dass** eine erhöhte Konzentration des Proteins mit der Bezeichnung Melanom inhibierende Aktivität (= MIA) im Serum oder Plasma nachgewiesen wird.

2. Verfahren gemäß Anspruch 1, **dadurch gekennzeichnet, dass** MIA immunologisch nachgewiesen wird.

3. Verfahren gemäß Anspruch 2, **dadurch gekennzeichnet, dass** monoklonale Antikörper gegen MIA verwendet werden.

4. Verfahren gemäß Anspruch 2 oder 3, **dadurch gekennzeichnet, dass** der Nachweis von MIA mittels eines heterogenen Immunoassays erfolgt.

5. Verfahren gemäß Anspruch 2 oder 3, **dadurch gekennzeichnet, dass** der Nachweis von MIA mittels eines homogenen Immunoassays erfolgt.

6. Verwendung von Antikörpern gegen MIA zur Diagnose von Knorpelerkrankungen.

## Claims

1. Process for diagnosing cartilage diseases, **characterized in that** an elevated level of Melanoma Inhibitory Activity (= MIA) protein is detected in the serum or plasma.

2. Process according to claim 1, **characterized in that** MIA is detected immunologically.

3. Process according to claim 2, **characterized in that** monoclonal antibodies against MIA are used.

4. Process according to claim 2 or 3, **characterized in that** detection of MIA is effected by means of a heterogeneous immunoassay.

5. Process according to claim 2 or 3, **characterized in that** detection of MIA is effected by means of a homogeneous immunoassay.

6. Use of antibodies against MIA for diagnosing cartilage diseases.

## Revendications

1. Procédé pour le diagnostic de maladies des cartilages **caractérisé en ce qu'**une concentration accrue de la protéine appelée activité inhibant les mélanomes (= MIA) est mise en évidence dans le sérum ou le plasma.

2. Procédé selon la revendication 1 **caractérisé en ce que** la MIA est mise en évidence par voie immunologique.

3. Procédé selon la revendication 2 **caractérisé en ce que** des anticorps monoclonaux contre la MIA sont utilisés.

4. Procédé selon la revendication 2 ou 3 **caractérisé en ce que** la mise en évidence de la MIA a lieu au moyen d'une immunoanalyse hétérogène.

5. Procédé selon la revendication 2 ou 3 **caractérisé en ce que** la mise en évidence de la MIA a lieu au moyen d'une immunoanalyse homogène.

6. Utilisation d'anticorps contre la MIA pour le diagnostic de maladies des cartilages.
